# EUROPEAN PATENT SPECIFICATION

(11) **EP 0 295 755 B1**
(45) Date of publication and mention of the grant of the patent: **16.03.1994**
(21) Application number: 88201235.4
(22) Date of filing: 16.06.1988
(51) Int. Cl.: A01K 43/00, G01N 33/08

(54) **A method for testing eggs for the existence of cracks or holes in the egg shells by examining the elasticity of said shells and an apparatus for carrying out said process**
Verfahren zum Überprüfen von Eiern auf das Vorhandensein von Rissen oder Löchern in den Eierschalen durch Prüfung der Elastizität jener Schalen und Vorrichtung zur Ausführung des Verfahrens
Méthode pour contrôler des oeufs sur l'existence des fissures ou des trous dans les coquilles d'oeufs par contrôle d'élasticité de celles-ci et dispositif de réalisation de cette méthode

(30) Priority: 18.06.1987 DK 3118/87
(43) Date of publication of application: 21.12.1988
(73) Proprietor: FPS Food Processing Systems B.V., NL-3771 VE Barneveld (NL)
(72) Inventor: Schouenborg, Kurt Otto Peter, DK-2610 Roedevro (DK)
(74) Representative: Smulders, Theodorus A.H.J., Ir.

(56) References cited:
- GB-A- 1 361 797
- US-A- 3 067 605
- US-A- 3 254 763
- US-A- 3 503 501

## Description

The present invention relates to a method for examining eggs to determine the presence of cracks or holes in the egg shell through the examination of the resiliency of said shell.

For many years, a great deal of work has been put into developing a suitable method and apparatus for the automatic detection of so-called cracked eggs, i.e. eggs having cracks or holes in their shells.

Such detection is important for several reasons. One is that the keeping quality of such eggs is reduced and their use as food may entail a health risk. Another reason is that damaged eggs cause loss and irritation in the further handling and distribution of these eggs.

Many attempts have been made to base such detection on the examination and measurement of egg shell characteristics in reaction to vibrations or oscillations applied to the egg shell. Such a method is described in US-A-3,503,501 in which a continuously vibrating probe is brought into contact with the egg, and the resistance and oscillations, as well as the phase differences between the oscillations of the probe and the oscillations produced in the egg shell are measured, while the egg is continuously rotated. In GB-A-1,361,797 a method is described in which the eggs are rolled over a number of piezoelectric crystals, thus receiving energy impulses. According to this method, the energy impulse returned by the eggs and the duration of that impulse is measured. The results of these measurements are used as a gauge for ascertaining the presence of cracks or holes in the shell. Another method is known from US-A-3,067,605, in which the rebound of light hammers striking passing eggs is measured. Eggs causing a rebound below a certain level are defined as cracked eggs.

None of these known methods has given satisfactory results in practice, so that it is still necessary to use the familiar method of inspecting the eggs as they pass through a candling station, in which a powerful light source is provided. Such a method is costly because of the wages involved and is also encumbered with the uncertainty involved in such enervating inspection work.

The object of the present invention is, therefore, to provide a method of the above-described type which is suitable for the fully-automatic detection of cracked eggs and which has a high degree of reliability.

The method according to the present invention is characterized by subjecting the egg to an examination cycle which consists in exercising intermittent pressure on at least a section of the egg shell by a movably mounted probe to create a series of in principle elastic deformations, where each subsequent deformation in the series is caused by the shell's action on the probe as a reaction to the immediately preceding deformation by subjecting the probe, from its original position at a distance from the shell, to a continuous force directed at the egg's shell, forcing the probe down to strike against the shell, and this results in an elastic deformation of same, whereupon the resilience of the egg shell causes it to return to its original shape, throwing back the probe, whereafter the probe, again as a result of the continuous force, is brought to strike against the shell once more before again being thrown back, repeating this action several times, with decreasing oscillations, so that the probe's action seen as a whole is a bouncing movement against the said egg shell, with decreasing oscillation, of which the amplitude and duration is measured, preferably electrically, and the number of oscillations of which the amplitude and duration exceed preset parameter values is counted and registered electronically, whereupon the above cycle is repeated a given number of times (n) and distributed evenly around the said egg's axis of symmetry, whereby the lowest registered number of oscillations from the (n) examination cycles is compared with an empirical parameter.

This method utilizes the fact that a section of an egg shell without cracks or holes has a higher degree of resiliency than a section with a crack or hole, and that this section, after being subjected to a certain degree of elastic deformation, will therefore generate both more and larger oscillations of the probe than will a section with cracks or holes. By the method described above, it has been found possible to achieve a degree of reliability which is fully equal to, and often considerably better than, the degree of reliability achieved by the traditional inspection of the eggs during their passage through a candling station. At the same time, the results of the measurements obtained by the above method can be read out by a computer, so that it is possible to obtain both an automatic and a direct indication of the shell quality of the individual egg, an indication which in turn can be used to control a sorting mechanism.

The above method is also suitable for use at very high speeds, so that it is applicable in inspection lines generally provided in egg packaging plants, in which eggs are continuously rotated around their axis of symmetry during the examination process.

When several parallel examinations are carried out concurrently, each on a different section of the shell along the axis of symmetry of the egg, and the number of oscillations of each individual section are counted and registered individually, the lowest value among the (n) examination cycles of all sections is compared with the empirical value referred to. Thus a particularly thorough examination of all sections of the shell can be achieved, so that there will be a high degree of certainty that any crack present in the shell will be revealed.

By the method according to this invention, it is possible to convert the damped oscillations of the probe into an electrical signal by means of a transducer consisting of a piezoelectric, optical, magnetic or capacitive device. This signal can then be converted by means of an amplifier, a comparator and a signal converter into rectangular digital impulses. These impulses are counted by a counting device and then transmitted to and stored in a computer, together with the results of other examinations of the egg. With the aid of a computer program it is then possible to ascertain the lowest value for the number of bouncing oscillations in an examination cycle, which value can be compared with an empirical parameter, characterizing an uncracked egg.

The invention also relates to an apparatus for carrying out the above-described method, this apparatus comprising
- revolving rollers (2, 3) for rotatably supporting the egg to be examined,
- at least one probe (6) for applying a force to the shell of the rotating egg (1), said force being capable of generating vibrations of the egg shell,
- transducer means (17) for sensing the force applied by the vibrating egg shell to the probe (6) and for producing electrical signals corresponding to the vibrations of the egg shell,
- means for comparing a representative value of said electrical signals with an empirical parameter, representing an uncracked egg.

Such apparatus is known from the above-mentioned US-A-3,503,501. The probe used in this apparatus comprises an electromechanical vibrator to produce oscillations therein and further an electromechanical transducer to determine the vibrations of the egg shell. The mass and volume of this probe is considerable. Due to its considerable mass, the probe remains in contact with the shell of the egg to be examined during the whole measuring period, and is not capable of making a bouncing movement with decreasing oscillations. Due to considerable probe volume only one section of the egg (near the mid plane thereof can be examined.

It is an object of the invention to provide an apparatus comprising one or more probes having a small mass and volume, these probes being capable of making a bouncing movement with decreasing oscillations, whose amplitude and duration are to be measured, the small volume of the probes enabling the simultaneous examination of an egg with several probes, each probe striking an egg on a different section thereof.

According to the invention, this object is achieved by providing an apparatus of the above type, wherein the probe is a substantially stiff, tube-shaped or rod-shaped element, one end thereof being elastically attached to a holder element, in which the transducer means are arranged and which is connected to a stationary base element by means of a leaf spring, an air chamber being arranged parallel to said leaf spring between opposing sides of the holder element and the base element, said air chamber being capable of moving the probe in the direction of an egg to be examined upon the introduction of pressurized air into said air chamber and of applying a bouncing force to the shell of said egg in order to cause a series of elastic deformations of said shell.

In another embodiment of the apparatus according to the invention, the probe is is a tube-shaped body, slidably mounted in a holder element which is vertically movable towards and away from an egg to be examined, which tube-shaped body, at the end directed towards said egg, is closed by a membrane to which a ball is fixed, the other end of said tube-shaped body being closed by a microphone, capable of sensing the vibrations, applied by the vibrating egg shell to the ball and of converting the vibrations sensed into electrical signals.

The method according to the invention and some embodiments of the apparatus for carrying out this method will now be described by way of example, with reference to the accompanying drawing, in which
Fig. 1 shows diagrammatically an apparatus according to the present invention;
Fig. 2 shows in more detail a first embodiment of a probe with its holder and driving mechanism;
Fig. 3 shows diagrammatically the position of the probe during the measuring process;
Fig. 4 shows a block diagram of an electronic signal processing device of the apparatus;
Fig. 5 shows an input signal of the comparator of the electronic signal processing device;
Fig. 6 shows the output signal of the comparator of the electronic signal processing device, being the converted input signal of Fig. 5;
Fig. 7 shows the output signal of the signal re-shaper, being the filtered output signal of Fig. 6;
Fig. 8 shows diagrammatically a second embodiment of a probe Fig. 9 is a variant of the probe shown in Fig. 8.

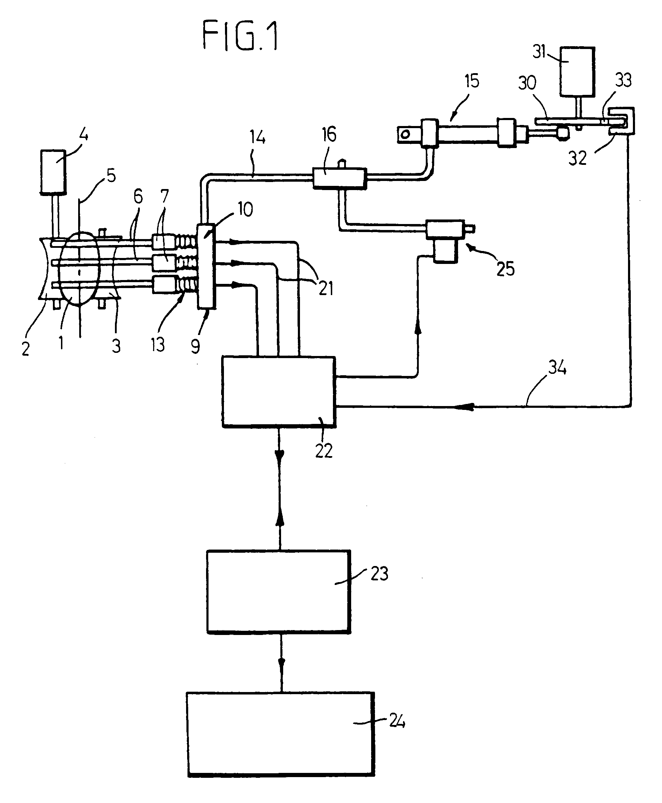

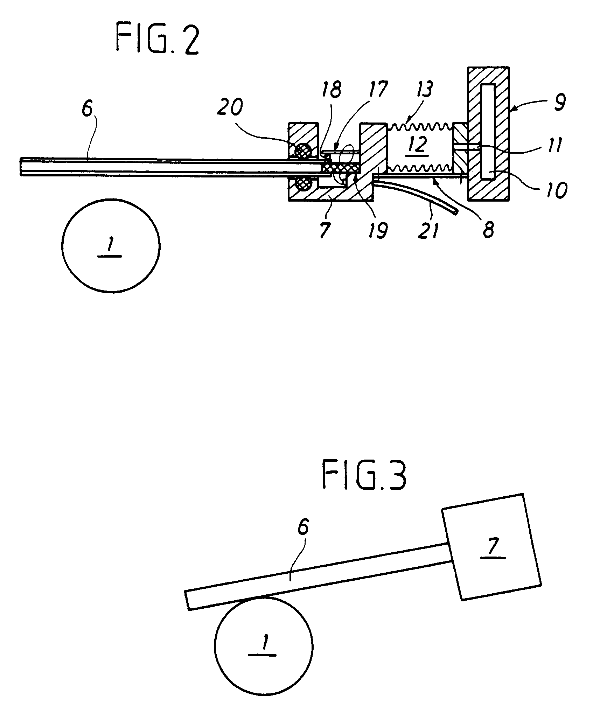

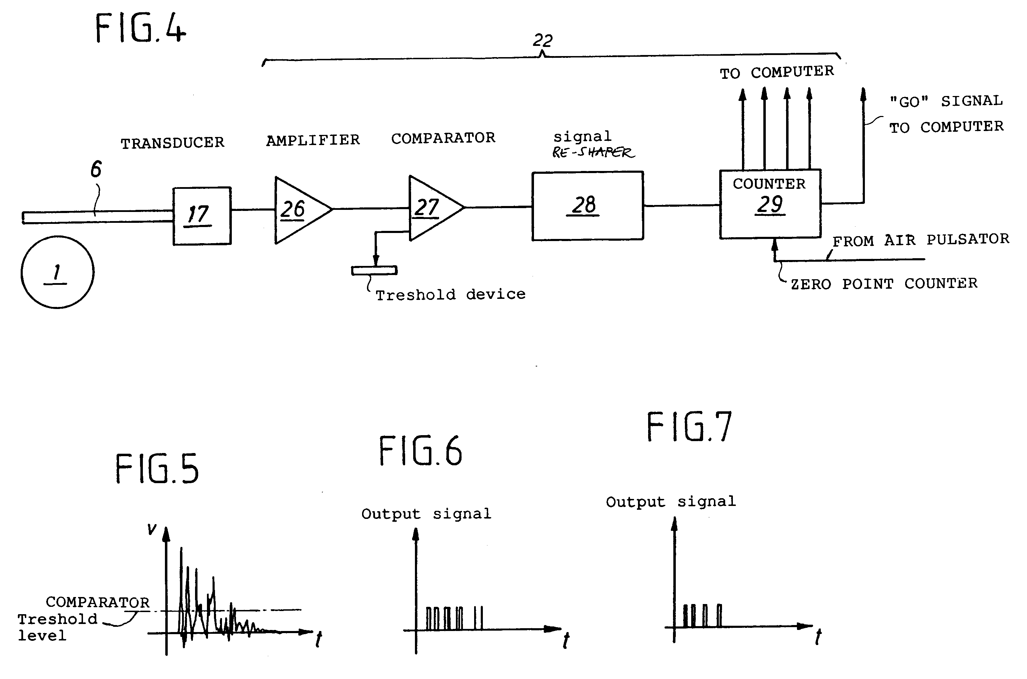

In Figures 1, 2 and 3, an apparatus according to this invention is shown adapted for the examination of a single egg 1. This apparatus comprises a base consisting of two closely placed, rotating rollers 2, 3 having parallel shafts which, by means of a motor 4, can be made to revolve in the same direction so that an egg 1 placed onto the rollers rotates mainly around its longitudinal axis of symmetry 5. Above the egg 1 there are arranged three probes 6 preferably glass tubes. Each glass tube 6 is at one end resiliently fixed into a holder 7, and this holder 7 is attached to a base 9 common to all three holders 7 by means of a leaf spring 8. The base 9 contains a chamber 10 for the distribution of compressed air, which chamber is connected through ducts 11 in the base 9 to individual air chambers 12, each belonging to its own holder 7. The individual chamber 12 extends between and is confined by the holder 7 and the base 9, a rubber bellows 13 being arranged between these elements 7, 9. The chamber 10 is connected to a compressed air source 15 by means of a pipe line 14 this air source 15 being shown here as an air pulsator, which is operated by a motor 31 via a crank disc 30. Inserted between the air pulsator 15 and the chaimber 10 for the distribution of compressed air is a vacuum escape valve 16. In the holder 7 for the glass tube 6, a transducer 17 in the form of a piezoelectric device is arranged, attached at one end to the holder 7. At the other end the transducer 17 is glued to the fixed end of the glass tube 6 by means of a silicone drop 18 so that the oscillating movement of the glass tube 6 causes deformations of the piezoelectric device 17. The glass tube 6 is resiliently mounted in its horizontal position, shown in Fig. 2, by means of a rubber chock 19 fitted to the holder 7 in which the glass tube 6 is fixed, and held flexibly in the holder 7 by means of two rubber chocks 20 so that, upon contact with the egg shell, it can swing in a plane that intersects the axis of symmetry 5 at a right angle. The upper and lower parts of the piezoelectric device 17 are connected to an electronic device 22 described in more detail hereafter. (See Fig. 4) by a two-core cable 21. This device 22, which contains a counter 29 for each glass tube 6, is connected to a computer 23 and a sorting mechanism 24. A yoke-shaped photoelectric cell 32 is placed over the crank disc 30 to cooperate with an air slot 33 in the disc 30 thus generating a signal to be transmitted to the electronic device 22 when the crank disc 30 is in a particular position. Further, a signal can be transmitted from the computer 23 via the electronic device 22 to a solenoid valve 25 which is connected to the vacuum escape valve 16.

When using the apparatus, the egg 1 is rotating around its axis of symmetry 5, and a signal is generated to close the solenoid valve 25. Compressed air is then fed from the compressed air source 15 through the pipe line 14 and into the chamber 10 for the distribution of compressed air. From here the air flows through the ducts 11 into the air chambers 12 connected to each of the three holders 7. The holders 7 with the glass tubes 6 will be placed in an inclined position as the leaf spring 8 is bent, so that the glass tubes 6 are pressed downwardly to strike against the surface of the egg 1 in the position shown in Fig. 3. The glass tube 6 is held under pressure against the egg as long as sufficient air pressure is present in the air chamber 12. The initial impact of the glass tube 6 against the egg will result in an elastic deformation of the egg shell. As a result of the resilience of the shell, this deformation is corrected, and the glass tube 6 is then thrown back before it is pressed down to strike against the egg shell once more as a result of the air pressure in the air chamber 12. This new blow again causes a deformation of the egg shell. This process is repeated so that the glass tube 6 carries out a bouncing movement against the egg shell. The number and impact of the bounces will depend on the resiliency of the shell. In other words, the glass tube 6 will bounce against the shell many times if the shell is whole, but only a few times if there is a crack in or near that section of the shell touched by the glass tube 6. The bouncing motion of the glass tube 6 is converted by the piezoelectric device 17 into an electrical signal which is related to the movements of the glass tube. This electrical signal generated by each of the three piezoelectric devices 17 is sent through the cables 21 to the electronic device 22 where these signals are processed. A more detailed description of this processing will be discussed hereafter, with reference to Figures 4-7, whereafter the resulting data are evaluated by the computer 23. After the measuring process has been concluded, the piston in the air pulsator 15 is moved, so that the air pressure in the pipe 14 is allowed to escape through the valve 16. In this way, the glass tubes 6 are raised in relation to the egg by means of the leaf spring 8. At the same time, the air slot 33 in the crank disc 30 is positioned in the yoke-shaped photoelectric cell 32, so that a signal is transmitted through the cable 34 to the electronic device 22 to reset the counter 29 to zero. The measurement process is then carried out a predetermined number of times (n) evenly distributed over the circumference of the egg, the glass tubes 6 being depressed each time to strike the surface of the rotating egg and to bounce off again. Each time three counters of the electronic device 22 register how many times the glass tubes 6 have bounced on the surface of the egg, the computer 23 being fed with the registered figures. Once the inspection has been completed, the solenoid valve 25 is connected to the atmosphere, and the measurement process is stopped. The total number of measurement results now contained in the computer's memory comprises 3 x n counter figures, which are then processed by the computer so that the lowest value is found and compared with a coded empirical parameter. If the resulting figure is lower than the parameter value, the egg is defined as a cracked egg, and this state is transferred in the form of a signal directly to a sorting mechanism 24 which automatically rejects the egg in question.

Figures 4-7 show the principle of how the electrical output signals of the transducer or piezoelectric device 17 are processed. These signals are transmitted through an amplifier 26 to a comparator 27, where the current oscillations, in principle sinus-shaped, are detected. An oscillation below a certain threshold, corresponding to the comparator threshold level shown in Fig. 5, is filtered out, while an oscillation above this threshold is transfered to a signal re-shaper 28 in the shape of digital impulses shown in Fig. 6, the length thereof corresponding to the length of the sinus oscillations at the comparator threshold level in Fig. 5, measured in the direction of the time axis t. In the signal re-shaper 28, which may be a one-shot multivibrator, the very short impulses in the output signal of the comparator 27 are filtered out so that the output signal of the re-shaper 28 is given the shape shown in Fig. 7, with the rectangular digital counter impulses only containing impulses of more than a given length. This signal is passed to a counter 29, which counts the number of impulses in the converted and reshaped signal. The counter 29 then transmits a "clear" signal to the computer 23, which subsequently reads out the counter 29. When the computer 23 has received all the results of the measurements, the solenoid valve 25 is opened, and the glass tubes 6 are then lifted off the shell of the egg.

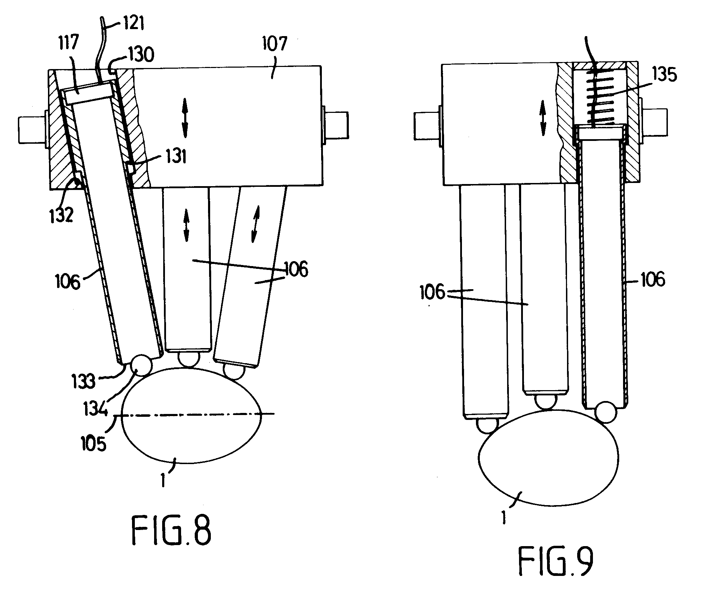

In the embodiments of Figures 8 and 9, an apparatus according to the invention is shown for the examination of a single egg 1. As the basic elements in these apparatuses are quite the same as in the apparatus described hereinbefore and shown in Figures 1, 2 and 4, the corresponding elements (i.e. elements which have the same function) will be indicated by the same reference numeral increased by 100.

The embodiments in Figs. 8 and 9 show a holder 107. This holder can be moved up and down by means not shown, such as a motor with an eccentric, a crank, or a pneumatic driven plunger. The holder 107 in this embodiment comprises three cavities 130, in which a tube 106 is movable. Of course the movement of the tube 106 in the cavity 130 is limited in the downward direction by stops 131 and 132.

The tube 106 which forms an air chamber, is closed at the upper side by a transducer, such as a microphone 117 and on the other side by a membrane 133, to which a ball 134 has been fixed. The microphone 117 is connected to a two-core cable 121 which leads to an electronic device 22 as described hereinbefore.

When using this apparatus, the egg 1 is made to rotate around its axis of symmetry 105 and the holder 107 is driven to make a vertical movement so that the tubes 106 with the balls 134 will be moved downwardly into contact with the egg shell. This will cause the air in the air chamber to vibrate. This vibration, picked up by the microphone 117 will be transmitted in the form of an electrical signal by the two-core cable 121 to the electronic device 22 hereinbefore described.

The difference between the apparatus shown in Figure 8 and in Figure 9 is mainly that all of the three tubes 106 in the embodiment of Fig. 9 are placed in a vertical position so that they are smoothly guided in the cavities 130. Further, the tubes 106 are influenced by a small spring 135, so that undesirable vibrations thereof will not occur.

The apparatus according to Fig. 9 provides a more reliable signal and further is easier to produce than an apparatus according to Fig. 8.

It may readily be understood that when eggs placed in several rows on an egg conveyor have to measured during transportation it will be possible to arrange a number of apparatuses as described above into an installation, in which the apparatuses can be moved individually, or in one or more groups along the path of transportation of the eggs over a certain distance. In this way each apparatus can carry out a complete measurement of a particular egg, before the apparatus is moved back to repeat the measuring process on a new egg on the conveyor.

## Claims

1. Method for the examination of eggs to determine the presence of cracks or holes in the egg shell through the examination of the resiliency of said shell, characterized by subjecting the egg (1) to an examination cycle which consists in exercising intermittent pressure on at least a section of the egg shell by a movably mounted probe (6; 106) to create a series of in principle elastic deformations, where each subsequent deformation in the series is caused by the shell's action on the probe (6; 106) as a reaction to the immediately preceding deformation; by subjecting the probe, from its original position at a distance from the shell, to a continuous force directed at the egg's shell, forcing the probe down to strike against the shell, and this results in an elastic deformation of same, whereupon the resilience of the egg shell causes it to return to its original shape, throwing back the probe, whereafter the probe, again as a result of the continuous force, is brought to strike against the shell once more before again being thrown back, repeating this action several times, with decreasing oscillations, so that the probe's action seen as a whole is a bouncing movement against the said egg shell with decreasing oscillation, of which the amplitude and duration is measured, preferably electrically, and the number of oscillations of which the amplitude and duration exceed preset parameter values is counted and registered electronically, whereupon the above cycle is repeated a given number of times (n) and distributed evenly around the said egg's axis of symmetry, the lowest registered number of oscillations from the (n) examination cycles being compared with an empirical parameter.

2. Method according to claim 1, characterized in that during the oscillations of the egg the rotation of the egg along its axis of symmetry (5; 105) is continued while the examinations are carried out.

3. Method according to claim 1 or 2, characterized by the concurrent examination of several sections of the egg's shell along the egg's axis of symmetry (5; 105), and the number of oscillations of each individual section being counted and registered individually, the lowest value among the (n) examination cycles multiplied by the number of sections being compared with said empirical value.

4. Method according to one or more of claims 1-3, characterized by the decreasing oscillations of the probe being converted into an electrical signal by means of a transducer (17; 117) comprising a piezoelectric, acoustic, optical, magnetic or a capacitive device, said signal being subsequently converted by means of an amplifier (26), a comparator (27) and a signal re-shaper (28) into rectangular digital impulses, said impulses being counted by a counting device (29) and then transmitted to and stored in a computer (23) together with the results of other examinations on the egg (1), the lowest value for the number of bouncing oscillations in an examination cycle being determined by means of a computer program and subsequently compared with said empirical parameter value.

5. Apparatus for carrying out the method according to one or more of the claims 1-4, said apparatus comprising
- revolving rollers (2, 3) for rotatably supporting the egg to be examined,
- at least one probe (6) for applying a force to the shell of the rotating egg (1), said force being capable of generating vibrations of the egg shell,
- transducer means (17) for sensing the force applied by the vibrating egg shell to the probe (6) and for producing electrical signals corresponding to the vibrations of the egg shell,
- means (22) for comparing a representative value of said electrical signals with an empirical parameter, representing an uncracked egg,
characterized in that said probe (6) is a substantially stiff, tube-shaped or rod-shaped element, one end thereof being elastically attached to a holder element (7), in which the transducer means (17) are arranged and which is connected to a stationary base element (9) by means of a leaf spring (8), an air chamber (12) being arranged parallel to said leaf spring (8) between opposing sides of the holder element (7) and the base element (9), said air chamber (12) being capable of moving the probe (6) in the direction of an egg to be examined upon the introduction of pressurized air into said air chamber (12) and of applying a bouncing force to the shell of said egg in order to cause a series of elastic deformations of said shell.

6. Apparatus according to claim 5, characterized in that several probes (6) are arranged in such a way that they are capable of simultaneously applying a bouncing force to the shell of an egg to be examined, each probe (6) striking said egg on a different section of said egg.

7. Apparatus for carrying out the method according to one or more of the claims 1-4, said apparatus comprising
- revolving rollers (2, 3) for rotatably supporting the egg to be examined,
- at least one probe (6) for applying a force to the shell of the rotating egg (1), said force being capable of generating vibrations of the egg shell,
- transducer means (17) for sensing the force applied by the vibrating egg shell to the probe (6) and for producing electrical signals corresponding to the vibrations of the egg shell,
- means (22) for comparing a representative value of said electrical signals with an empirical parameter, representing an uncracked egg,
characterized in that said probe (106) is a tube-shaped body (106), slidably mounted in a holder element (107) which is vertically movable towards and away from an egg to be examined, which tube-shaped body (106), at the end directed towards said egg, is closed by a membrane (133) to which a ball (134) is fixed, the other end of said tube-shaped body (106) being closed by a microphone (117), capable of sensing the vibrations, applied by the vibrating egg shell to the ball (134) and of converting the vibrations sensed into electrical signals.

8. Apparatus according to claim 7, characterized in that several tube-shaped bodies (106) are mounted in said holder element (107) in such a way that they are capable of simultaneously applying a bouncing force to the shell of an egg to be examined, each ball (134) striking said egg on a different section of said egg.

## Patentansprüche

1. Verfahren zur Überprüfung von Eiern auf das Vorhandensein von Rissen oder Löchern in den Eierschalen durch Prüfung der Elastizität jener Schalen, dadurch gekennzeichnet, daß das Ei (1) einem Prüfungszyklus unterzogen wird, der darin besteht, daß durch eine beweglich montierte Sonde (6; 106) ein intermittierender Druck auf mindestens eine Sektion der Eierschale ausgeübt wird, um eine Reihe von im wesentlichen elastischen Deformationen zu erzeugen, wobei jede folgende Deformation in der Reihe durch die Einwirkung der Schale auf die Sonde (6; 106) als Reaktion auf die unmittelbar vorhergehende Deformation verursacht wird; die Sonde, von ihrer Ausgangslage im Abstand von der Schale, einer auf die Eierschale gerichteten kontinuierlichen Kraft unterzogen wird, wobei die Sonde heruntergedrückt wird, um an die Schale anzustoßen, was in eine elastische Deformation derselben resultiert, worauf die Elastizität der Eierschale dieselbe in ihre Ursprungsform zurückkehren läßt, während die Sonde zurückgeworfen wird, worauf die Sonde, wiederum infolge der kontinuierlichen Kraft, noch einmal an die Schale anstößt, bevor sie wieder zurückgeworfen wird, welche Handlung mehrmals wiederholt wird, während die Oszillationen abnehmen, so daß die Wirkung der Sonde in ihrer Gesamtheit gesehen eine federnde Bewegung gegen die genannte Eierschale ist, während die Oszillation abnimmt, deren Amplitude und Dauer, vorzugsweise elektrisch, gemessen werden, und die Anzahl Oszillationen, deren Amplitude and Dauer vorgegebene Parameterwerte übersteigt, gezählt und elektronisch registriert wird, worauf der obengenannte Zyklus eine gegebene Anzahl Male (n) wiederholt und gleichmäßig um die Symmetrieachse des genannten Eies verteilt wird, wobei die niedrigst registrierte Anzahl Oszillationen aus den (n) Prüfungszyklen mit einem empirischen Parameter verglichen wird.

2. Verfahren nach Anspruch 1, dadurch gekennzeichnet, daß während der Oszillationen des Eies die Drehung des Eies um seine Symmetrieachse (5; 105) fortgesetzt wird, während die Prüfungen durchgeführt werden.

3. Verfahren nach Anspruch 1 oder 2, dadurch gekennzeichnet, daß gleichzeitig mehrere Sektionen der Eierschale um die Symmetrieachse (5; 105) des Eies geprüft werden, wobei die Anzahl Oszillationen jeder einzelnen Sektion gezählt und einzeln registriert wird und der niedrigste Wert unter den (n) Prüfungszyklen, multipliziert mit der Anzahl Sektionen, mit dem genannten empirischen Wert verglichen wird.

4. Verfahren nach einem oder mehreren der Ansprüche 1 bis 3, dadurch gekennzeichnet, daß die abnehmenden Oszillationen der Sonde durch einen Transducer (17; 117), der eine piezoelektrische, akustische, optische, magnetische oder kapazitive Einrichtung enthält, in ein elektrisches Signal umgewandelt werden, welches Signal danach durch einen Verstärker (26), einen Komparator (27) und einen Signalumformer (28) in rechteckige digitale Impulse umgewandelt wird, welche Impulse durch eine Zähleinrichtung (29) gezählt und dann in einen Computer (23) überführt und darin gespeichert werden, zusammen mit den Resultaten anderer Prüfungen am Ei (1), wobei der niedrigste Wert für die Anzahl federnder Oszillationen in einem Prüfungszyklus durch ein Computerprogramm bestimmt und danach mit dem genannten empirischen Parameterwert verglichen wird.

5. Vorrichtung zur Durchführung des Verfahrens nach einem oder mehreren der Ansprüche 1 bis 4, enthaltend
- drehbare Walzen (2, 3) zur drehbaren Unterstützung des zu prüfenden Eies,
- mindestens eine Sonde (6) zur Ausübung einer Kraft auf die Schale des drehenden Eies (1), welche Kraft Vibrationen der Eierschale erzeugen kann,
- einen Transducer (17) zur Abtastung der durch die vibrierende Eierschale auf die Sonde (6) ausgeübten Kraft und zur Erzeugung von elektrischen Signalen, die den Vibrationen der Eierschale entsprechen,
- Mittel (22) zur Vergleichung eines repräsentativen Wertes der genannten elektrischen Signale mit einem empirischen Parameter, der ein rißfreies Ei repräsentiert,
dadurch gekennzeichnet, daß die Sonde (6) ein im wesentlichen starres, rohr- oder stabförmiges Element ist, dessen eines Ende elastisch an einem Halterelement (7) befestigt ist, in dem der Transducer (17) angeordnet ist und das durch eine Blattfeder (8) mit einem ortsfesten Basiselement (9) verbunden ist, wobei eine Luftkammer (12) parallel zur Blattfeder (8) zwischen gegenüberliegenden Seiten des Halterelements (7) und des Basiselements (9) angeordnet ist, welche Luftkammer (12) die Sonde (6) in Richtung auf ein zu prüfendes Ei bewegen kann, wenn Druckluft in die Luftkammer (12) eingeführt wird und eine federnde Kraft auf die Schale des genannten Eies ausgeübt wird, um eine Reihe elastischer Deformationen jener Schale zu erzeugen.

6. Vorrichtung nach Anspruch 5, dadurch gekennzeichnet, daß mehrere Sonden (6) derart angeordnet sind, daß sie gleichzeitig eine federnde Kraft auf die Schale eines zu prüfenden Eies ausüben können, wobei jede Sonde (6) das genannte Ei an einer verschiedenen Sektion jenes Eies anstößt.

7. Vorrichtung zur Durchführung des Verfahrens nach einem oder mehreren der Ansprüche 1 bis 4, enthaltend
- drehbare Walzen (2, 3) zur drehbaren Unterstützung des zu prüfenden Eies,
- mindestens eine Sonde (6) zur Ausübung einer Kraft auf die Schale des drehenden Eies (1), welche Kraft Vibrationen der Eierschale erzeugen kann,
- einen Transducer (17) zur Abtastung der durch die vibrierende Eierschale auf die Sonde (6) ausgeübten Kraft und zur Erzeugung von elektrischen Signalen, die den Vibrationen der Eierschale entsprechen,
- Mittel (22) zur Vergleichung eines repräsentativen Wertes der genannten elektrischen Signale mit einem empirischen Parameter, der ein rißfreies Ei repräsentiert,
dadurch gekennzeichnet, daß die Sonde (106) ein rohrförmiger Körper (106) ist, der gleitend in einem Halterelement (107) montiert ist, das vertikal auf ein zu prüfendes Ei zu und von diesem weg bewegbar ist, welcher rohrförmige Körper (106) am gegen das genannte Ei gerichteten Ende durch eine Membrane (133) geschlossen ist, an der eine Kugel (134) befestigt ist, wobei das andere Ende des genannten rohrförmigen Körpers (106) durch ein Mikrophon (117) geschlossen ist, das die von der vibrierenden Eierschale auf die Kugel (134) ausgeübten Vibrationen abtasten und die abgetasteten Vibrationen in elektrische Signale umwandeln kann.

8. Vorrichtung nach Anspruch 7, dadurch gekennzeichnet, daß mehrere rohrförmige Körper (106) im Halterelement (107) derart montiert sind, daß sie gleichzeitig eine federnde Kraft auf die Schale eines zu prüfenden Eies ausüben können, wobei jede Kugel (134) das genannte Ei an einer verschiedenen Sektion jenes Eies anstößt.

## Revendications

1. Procédé d'examen d'oeufs, en vue de déterminer la présence de fissures ou de trous dans la coquille d'oeuf, par examen de l'élasticité de ladite coquille, caractérisé en ce que l'on soumet l'oeuf (1) à un cycle d'examen qui consiste à exercer une pression intermittente sur au moins une partie de la coquille d'oeuf à l'aide d'une sonde (6; 106) montée à déplacement, en vue de créer une série de déformations en principe élastiques, dans laquelle chaque déformation suivante de la série est provoquée par l'action de la coquille sur la sonde (6; 106) en réaction à la déformation immédiatement antérieure; en ce qu'à partir de la position initiale de la sonde à distance de la coquille, on soumet la sonde à une force continue dirigée vers la coquille d'oeuf, en forçant la sonde à venir frapper contre la coquille, ce qui entraîne une déformation élastique de cette dernière, suite à quoi l'élasticité de la coquille d'oeuf provoque son retour vers sa forme initiale, ce qui renvoie la sonde, suite à quoi, à nouveau en conséquence de la force continue, la sonde est amenée à frapper à nouveau la coquille avant d'être à nouveau renvoyée en arrière, cette action étant répétée plusieurs fois avec des oscillations décroissantes, de telle sorte que vue globalement l'action de la sonde est un mouvement de rebond à oscillations décroissantes contre ladite coquille d'oeuf, oscillations dont l'amplitude et la durée sont mesurées, de préférence électriquement, le nombre d'oscillations dont l'amplitude et la durée excèdent des valeurs pré-réglées de paramètres étant compté et enregistré électroniquement, suite à quoi le cycle ci-dessus est répété un certain nombre (n) de fois et en distribution régulière autour de l'axe de symétrie dudit oeuf, le nombre d'oscillations le plus bas enregistré à partir des (n) cycles d'examen étant comparé à un paramètre empirique.

2. Procédé selon la revendication 1, caractérisé en ce qu'au cours des oscillations de l'oeuf, on poursuit la rotation de l'oeuf autour de son axe de symétrie (5; 105) pendant que l'on effectue les examens.

3. Procédé selon la revendication 1 ou 2, caractérisé par l'examen simultané de plusieurs parties de la coquille d'oeuf le long de l'axe de symétrie (5; 105) de l'oeuf, le nombre d'oscillations de chaque partie individuelle étant compté et enregistré individuellement, la valeur la plus basse parmi les (n) cycles d'examen, multipliée par le nombre de parties, étant comparée à ladite valeur empirique.

4. Procédé selon l'une ou plusieurs des revendications 1 - 3, caractérisé en ce que les oscillations décroissantes de la sonde sont converties en un signal électrique au moyen d'un transducteur (17; 117) comportant un dispositif piézo-électrique, acoustique, optique, magnétique ou capacitif, ledit signal étant ensuite converti au moyen d'un amplificateur (26), d'un comparateur (27) et d'un dispositif (28) de mise en forme du signal en des impulsions numériques rectangulaires, lesdites impulsions étant comptées par un dispositif de comptage (29) et ensuite transmises à un ordinateur (23) dans lequel elles sont mises en mémoire, de même que les résultats d'autres examens de l'oeuf (1), la valeur la plus basse du nombre d'oscillations de rebond d'un cycle d'examen étant déterminée au moyen d'un programme informatique et ensuite comparée à ladite valeur empirique du paramètre.

5. Appareil en vue de mettre en oeuvre le procédé selon l'une ou plusieurs des revendications 1 - 4, ledit appareil comprenant:
- des rouleaux rotatifs (2, 3) pour soutenir à rotation l'oeuf à examiner,
- au moins une sonde (6) en vue d'appliquer une force sur la coque de l'oeuf (1) en rotation, ladite force étant capable de créer des vibrations de la coquille d'oeuf,
- des moyens transducteurs (17) en vue de détecter la force appliquée par la coquille d'oeuf en vibration à la sonde (6) et de produire des signaux électriques correspondant aux vibrations de la coquille d'oeuf,
- des moyens (22) en vue de comparer une valeur représentative desdits signaux électriques avec un paramètre empirique représentant un oeuf non fendillé,
caractérisé en ce que ladite sonde (6) est un élément essentiellement rigide, en forme de tube ou de barre, dont une extrémité est fixée élastiquement à un élément porteur (7) dans lequel les moyens transducteurs (17) sont agencés et qui est relié à un élément stationnaire de base (9) au moyen d'un ressort à lame (8), une chambre à air (12) étant agencée en parallèle audit ressort à lame (8), entre des côtés opposés de l'élément porteur (7) et l'élément de base (9), ladite chambre à air (12) étant capable de déplacer la sonde (6) dans la direction d'un oeuf à examiner, lorsque l'on introduit de l'air sous pression dans ladite chambre à air (12), et étant capable d'appliquer une force de rebond à la coquille dudit oeuf, en vue de provoquer une série de déformations élastiques de ladite coquille.

6. Appareil selon la revendication 5, caractérisé en ce que plusieurs sondes (6) sont agencées de telle sorte qu'elles sont capables d'appliquer simultanément une force de rebond à la coquille d'un oeuf à examiner, chaque sonde (6) frappant ledit oeuf sur une partie différente dudit oeuf.

7. Appareil en vue de mettre en oeuvre le procédé selon l'une ou plusieurs des revendications 1-4, ledit appareil comprenant:
- des rouleaux rotatifs (2, 3) pour soutenir à rotation l'oeuf à examiner,
- au moins une sonde (6) en vue d'appliquer une force sur la coquille de l'oeuf (1) en rotation, ladite force étant capable de créer des vibrations de la coquille d'oeuf,
- des moyens transducteurs (17) en vue de détecter la force appliquée par la coquille d'oeuf en vibration à la sonde (6) et de produire des signaux électriques correspondant aux vibrations de la coquille d'oeuf,
- des moyens (22) en vue de comparer une valeur représentative desdits signaux électriques avec un paramètre empirique représentant un oeuf non fendillé,
caractérisé en ce que ladite sonde (106) est un corps (106) en forme de tube monté à coulissement dans un élément porteur (107) qui peut être déplacé verticalement dans les deux sens par rapport à un oeuf à examiner, ledit corps (106) en forme de tube étant refermé à l'extrémité dirigée vers ledit oeuf par une membrane (133) sur laquelle une bille (134) est fixée, l'autre extrémité dudit corps (106) en forme de tube étant refermée par un microphone (117) capable de détecter les vibrations appliquées par la coquille d'oeuf en vibration à la bille (134) et de convertir les vibrations détectées en signaux électriques.

8. Appareil selon la revendication 7, caractérisé en ce que plusieurs corps (106) en forme de tube sont montés dans ledit élément porteur (107) de telle sorte qu'ils sont capables d'appliquer simultanément une force de rebond sur la coquille d'un oeuf à examiner, chaque bille (134) frappant ledit oeuf sur une partie différente dudit oeuf.
